# EUROPEAN PATENT APPLICATION

(11) **EP 1 767 190 A2**
(43) Date of publication of application: **28.03.2007**
(21) Application number: 06291456.9
(22) Date of filing: 18.09.2006
(51) Int. Cl.: A61K 8/86, A61K 8/39, A61Q 5/00

(54) **Anhydrous hair compositions comprising a phosphate ester**

(30) Priority: 23.09.2005 US 233836
(71) Applicant: L'Oreal, 75008 Paris (FR)
(72) Inventor: Ellington, Angela, Flossmoor, IL 60422 (US); Hunter, Nikisha, Chicago, IL 60628 (US)
(74) Representative: Dossmann, Gérard

(57) **Abstract**

A composition and process for treating a keratinous substrate involving contacting the keratinous substrate with a substantially anhydrous composition containing: (a) at least one alkoxylated alkyl phosphate ester; (b) at least one ester oil component; and (c) optionally, at least one non-ester oil component.

## Description

### BACKGROUND OF THE INVENTION

The present invention generally relates to a composition and process for treating hair based on an anhydrous composition that provides product rinseability, spreadability and cosmetic feel.

Hair care compositions such as pomades, for example, are usually made up of mineral oil and petrolatum and are used to enhance the feel and appearance of hair. With repeated use, however, these materials tend to leave an oily buildup and weigh hair down. Moreover, due to their high oil content, products of this nature tend to be difficult to rinse out of the hair.

A need therefore exists for a hair treatment composition and process which offers exceptional cosmetic feel plus improved product distribution and rinseability.

### BRIEF SUMMARY OF THE INVENTION

The present invention relates to a cosmetic composition containing:
(a) at least one alkoxylated alkyl phosphate ester;
(b) at least one ester oil component; and
   optionally, at least one non-ester oil component; such composition being substantially anhydrous.
   The present invention also relates to a process for treating a keratinous substrate involving contacting the substrate with the above-disclosed cosmetic composition.

### DETAILED DESCRIPTION OF THE INVENTION

Other than in the operating examples, or where otherwise indicated, all numbers expressing quantities of ingredients and/or reaction conditions are to be understood as being modified in all instances by the term "about".

The term "substantially anhydrous" means that the composition is either completely free of water or contains no appreciable amount of water, preferably no more than 5% by weight, and more preferably no more than 1% by weight, based on the weight of the composition.

The cosmetic compositions and methods of the present invention can comprise, consist of, or consist essentially of the essential elements and limitations of the invention described herein, and may comprise any additional or optional ingredients described herein or otherwise useful in personal care compositions intended for application to the hair.

The present invention relates to a cosmetic composition, substantially non-aqueous, for use in treating a keratinous substrate such as hair. The cosmetic composition is based on a mixture of at least one alkoxylated alkyl phosphate ester and optionally a treating component containing at least one ester oil and a saturated and/or unsaturated oil.

Alkoxylated Alkyl Phosphate Ester

The alkoxylated alkyl phosphate ester of the present invention preferably corresponds to the general formula:

R₁-(OCH₂ CH₂)ₙ(OCH(CH₃)CH₂)ₘ - OPO-(OH)₂

wherein R₁ is an alkyl group having from 8 to 40 carbon atoms, preferably from 10 to 22 carbon atoms, and more preferably from 12 to 18 carbon atoms, n is a number from 1 to 40, preferably from 2 to 24, and more preferably from 10 to 18, and m is a number from 0 to 20. The preferred alkyl phosphate ester comprises 5 moles of propylene oxide and 10 moles of ethylene oxide. In general, the lower the number of carbon atoms in the alkyl group R₁ of the alkoxylated alkyl phosphate ester, the more irritating to the skin and the less soluble in water the phosphate ester becomes. In contrast, the higher the number of carbon atoms in the alkyl group, the milder to the skin and the thicker and more waxy the resultant product becomes. Accordingly, for best results, R₁ should have from 14 to 18 carbon atoms.

The alkoxylated alkyl phosphate ester is preferably present in the present composition in an amount of from greater than 0% to 10% by weight, such as greater than 0 to 5% by weight, for example greater than 0 to 3% by weight, based on the total weight of the composition. A particularly preferred alkoxylated alkyl phosphate ester for use in the present invention is PPG-5-ceteth-10 phosphate, sold by Croda as CRODAFOS SG^{®}.

Ester Oil Component

Suitable ester oils which may be employed can be chosen from mono-, di- and tri- esters of glycerol. Preferred glyceride fatty esters are derived from carboxylic acids of carbon chain length ranging from C₆ to C₄₀, preferably from C₁₀ to C₂₂, and more preferably from C₁₂ to C₁₈.

Synthetic ester oils which may be used include, but are not limited to, trimyristin, triolein, tristearin and glyceryl dilaurate. Vegetable derived glyceride fatty esters are particularly preferred, and specific examples of preferred materials as sources of glyceride fatty esters include peanut oil, sesame oil, avocado oil, coconut, cocoa butter, almond oil, safflower oil, corn oil, cotton seed oil, castor oil, hydrogenated castor oil, olive oil, jojoba oil, palm oil, soybean oil, wheat germ oil, linseed oil, and sunflower seed oil. Coconut oil, sunflower oil, castor oil and mixtures thereof are particularly preferred.

Particularly useful glyceryl ester oils herein include caprylic/capric triglyceride with the tradename Miglyol812, from Degussa-Huls A G (Frankfurt, Germany), triisostearin with tradename SUN ESPOL G-318 available from Taiyo Kagaku, triolein with tradename CITHROL GTO available from Croda, Inc. (Parsippany, N.J., USA), trilinolein with tradename EFADERMA-F available from Vevy (Genova, Italy), or tradename EFA-GLYCERIDES from Brooks (South Plainfield, N.J., USA).

Additional examples of suitable esters include, for example, C12-15 alkyl benzoate (such as FINSOLV TN from Finetex, Elmwood Park, N.J.); octyl methoxy cinnamate (such as ESCALOL 557 from ISP, Wayne, N.J. (but in amounts less than 6% because of irritancy); isostearyl isostearate (such as (PRISORINE IS 2039 from Unichema, Chicago, I11.); benzyl benzoate; 2,6-di-(ethylhexyl)naphthalate (such as Hallbrite TQ from the C.P. Hall Company, Bedford Park, I11.); butyl octyl salicylate; glyceryl monostearate; n-dibutyl sebacate; isopropyl myristate; isopropyl palmitate; butyl stearate; cetyl lactate; isocetyl stearate; hexyl laurate; decyl oleate; isostearyl isostearate; ethyl hexyl maleate; sorbitan monoaurate; sorbitan monooleate; sorbitan sesquioleate; sorbitan trioleate; isopropyl palmitate; isopropyl stearate; stearyl stearate; diisopropyl adipate; diisopropyl sebacate; butyl myristate; isopropyl laurate; isotridecyl isononanoate; isostearyl neopentanoate; tridecyl neopentanoate; cetyl octanoate; cetyl ricinoleate; decyl isostearate; isodecyl oleate; isodecyl neopentanoate; isohexyl neopentanoate; tridecyl octanoate; alkyl benzoate; propyl myristate; propyl palmitate; tridecyl stearate; octyl palmitate and so on. Other suitable esters may also include esters such as tridecyl trimellilate, neopentyl glycol dicaprilate/dicaprate.

Also suitable may be synthetic or semi-synthetic glyceryl esters, e.g. fatty acid mono-, di-, and triglycerides which are natural fats or oils that have been modified, for example, acetylated castor oil, glyceryl stearate, glyceryl dioleate, glyceryl distearate, glyceryl trioctanoate, glyceryl linoleate, glyceryl myristate, glyceryl isostearate, PEG castor oils, PEG glyceryl oleates, PEG glyceryl stearates, PEG glyceryl tallowates, and so on.

The at least one ester oil component is preferably employed in the present composition in an amount of from greater than 0 to 95% by weight, based on the total weight of the composition. According to the present invention, the composition can contain high amounts of ester oil component(s), such as greater than 80 % by weight, as well as low amounts of ester oil component(s), such as greater than 0 to 10% by weight, or even greater than 0 to 5% by weight, based on the total weight of the composition. According to a preferred embodiment , the at least one ester oil component is employed in the present composition in an amount of from 25 to 95% by weight, more preferably from 25 to 50% by weight, based on the total weight of the composition. A particularly preferred ester oil component for use in the present invention is a triglyceride, and more particularly, caprylic/capric triglyceride.

Non-ester Oil Component

The at least one non-ester oil component may be chosen from natural, synthetic, saturated and unsaturated oils, but preferably not mineral oils. Mention may be made of linear, branched and/or cyclic alkanes which may be volatile and, in particular, liquid paraffin, liquid petroleum jelly or hydrogenated polyisobutylene, isododecane or "Isopars", volatile isoparaffins.

Mention may also be made of aliphatic fatty liquid monoalcohols containing 6 to 40 carbon atoms, the hydrocarbon-based chain not comprising a substitution group. Monoalcohols according to the invention that may be mentioned include oleyl alcohol, decanol, octyldodecanol and linoleyl alcohol.

Mention may also be made of silicone oils such as polydimethylsiloxanes and polymethylphenylsiloxanes, optionally substituted with aliphatic and/or aromatic groups, which are optionally fluorinated, or with functional groups such as hydroxyl, thiol and/or amine groups, and volatile silicone oils, which are especially cyclic.

In particular, mention may be made of volatile and/or non-volatile, optionally branched silicone oils. The term "volatile oil" means an oil capable of evaporating from the skin or the lips in less than one hour, and especially having a vapor pressure, at room temperature and atmospheric pressure, ranging from 10⁻³ to 300 mmHg (0.13 Pa to 40,000 Pa).

As volatile silicone oils that may be used in the invention, mention may be made of linear or cyclic silicones containing from 2 to 7 silicon atoms, these silicones optionally comprising alkyl or alkoxy groups containing from 1 to 10 carbon atoms. Mention may be made in particular of octamethylcyclotetrasiloxane, decamethylcyclopentasiloxane, dodecamethylcyclohexasiloxane, heptamethylhexyltrisiloxane, heptamethyloctyltrisiloxane, octamethyltrisiloxane and decamethyltetrasiloxane, and mixtures thereof.

Among the non-volatile silicone oils that may be mentioned are non-volatile polydialkylsiloxanes, such as non-volatile polydimethylsiloxanes (PDMS); polydimethylsiloxanes comprising alkyl, alkoxy or phenyl groups, which are pendent or at the end of a silicone chain, these groups containing from 2 to 24 carbon atoms; phenyl silicones, for instance phenyl trimethicones, phenyl dimethicones, phenyl trimethylsiloxy diphenylsiloxanes, diphenyl dimethicones, diphenyl methyldiphenyltrisiloxanes and polymethyl-phenylsiloxanes; polysiloxanes modified with fatty acids (especially of C₈-C₂₀), fatty alcohols (especially of C₈-C₂₀) or polyoxyalkylenes (especially polyoxy-ethylene and/or polyoxypropylene); amino polysiloxanes; polysiloxanes containing hydroxyl groups; fluoro poly-siloxanes comprising a fluorinated group that is pendent or at the end of a silicone chain, containing from 1 to 12 carbon atoms, all or some of the hydrogen atoms of which are replaced with fluorine atoms; and mixtures thereof.

The at least one non-ester oil component may be employed in the present composition in an amount of: up to 95% by weight; up to 50% by weight; up to 25% by weight; up to 15% by weight; up to 10% by weight; up to 5% by weight, based on the total weight of the composition.

It should be noted that while the inclusion of a mineral oil is not recommended due to the drawbacks of poor rinseability and build-up, minor amounts of these oils may nevertheless be employed without departing from the spirit of the invention.

Adjuvants

The compositions of the present invention may also contain adjuvants suitable for hair care. Suitable hair care adjuvants include, but are not limited to:

(i) Natural hair root nutrients, such as amino acids and sugars. Examples of suitable amino acids include arginine, cysteine, glutamine, glutamic acid, isoleucine, leucine, methionine, serine and valine, and/or precursors and derivatives thereof. The amino acids may be added singly, in mixtures, or in the form of peptides, e.g. di- and tripeptides. The amino acids may also be added in the form of a protein hydrolysate, such as a keratin or collagen hydrolysate.
Suitable sugars are glucose, dextrose and fructose. These may be added singly or in the form of, e. g. fruit extracts. A particularly preferred combination of natural hair root nutrients for inclusion in compositions of the invention is isoleucine and glucose. A particularly preferred amino acid nutrient is arginine.

(ii) Hair fiber benefit agents. Examples are:
ceramides, for moisturizing the fiber and maintaining cuticle integrity. Ceramides are available by extraction from natural sources, or as synthetic ceramides and pseudoceramides. A preferred ceramide is Ceramide II, ex Quest. Mixtures of ceramides may also be suitable, such as Ceramides LS, ex Laboratories Serobiologiques.

Nonvolatile silicone compounds may also be employed as adjuvants in the present invention such as, for example, a polydimethylsiloxane, polyalkyl siloxane, a polyaryl siloxane or a polyalkylaryl siloxane. The nonvolatile silicones are nonfunctional siloxanes or siloxane mixtures having a viscosity of about 10 to about 10, 000 cst , and most preferred viscosity about 10 to 500 cst at 25° C. A nonvolatile silicone compound is described as having a boiling point at atmospheric pressure of greater than about 250° C. A particularly preferred nonvolatile silicone compound is cyclopentasiloxane, commercially available from Dow Corning under the tradename DC 245.

Additional adjuvants include gelling agents, waxes, preservatives, suspending agents, volatile solvents, thickening agents, film formers, spreading agent, dispersants, antifoaming agents, wetting agents, UV-screening agents, antioxidants, perfumes, fillers, active agents, moisturizers, vitamins, biological materials, and derivatives of any of the foregoing.

The composition of the present invention possesses both good spreadability with regards to the treating component, and good rinseability with regards to build-up inhibition.

According to another embodiment of the present invention, there is also provided a process for treating a keratinous substrate, such as hair, involving contacting the substrate with the above-disclosed composition. The process facilitates the styling of substrates, such as hair, in a cosmetically appealing manner facilitated by the good spreadability and rinsability of the above-disclosed composition.

The following examples further describe and demonstrate embodiments within the scope of the present invention. The examples are given solely for the purpose of illustration and are not to be construed as limitations of the present invention, as many variations thereof are possible without departing from the spirit and scope of the invention. All exemplified amounts are concentrations by weight of the total composition, unless otherwise specified.

### EXAMPLES

Base Formulation:

| INCI Name | Trade Name | %w/w |
|---|---|---|
| Caprylic/Capric Triglyceride | Neobee M5 | 47.70 |
| Isopropyl Myristate | IPM | 25.60 |
| C12-15 Alkyl Benzoate | Finsolv TN | 2.00 |
| Palm Kernel Oil | | 13.25 |
| C18-36 Triglyceride | Synchrowax HGLC | 6.00 |
| Paraffin | Aristowax 143 | 4.00 |
| Propylparaben | | 0.10 |
| BHT | Nipanox BHT | 0.05 |
| Cyclopentasiloxane | DC245 | 1.00 |
| Fragrance | | 0.30 |
| Total | | 100.00 |

Two (2) 200g samples of the base were placed in a container. Into one container were added various amounts of a non-phosphate ester, specifically, PPG-5-Ceteth-20 (PROCETYL AWS^{®}). Into the other container were added various amounts of a phosphate ester in accordance with the present invention, specifically, PPG-5-Ceteth-10 Phosphate (CRODAFOS SG^{®}), for comparison purposes. Each was then tested to determine its spreadability by applying it manually onto hair tresses.

Spreadability measurements are subjective intensity measurements based on a continuum anchored at one side with Lanolin at 0.2, which does not spread easily and at the other with baby oil at 9.7, which spreads easily. Petrolatum at 2.9 spreads better than Lanolin, and Vaseline Intensive Care at 6.9, spreads better than Petrolatum, but not as well as baby oil (See Inolex Technical Report No. 25, entitled LEXOL^{®} Emollients: A basic Guide to INOLEX^{®} Ester Selection). Spreadability was evaluated on a scale of 1-10 with 10 being the best spreadability. The results are found in Table I, below.

**Table I**

| Amount of Ester present in base | 0% | 0.5% | 1.0% | 2.0% | 3.0% |
|---|---|---|---|---|---|
| CRODAFOS SG score | 5.4 | 7.4 | 8.9 | 9.5 | 9.3 |
| PROCETYL AWS score | 5.4 | 5.7 | 5.9 | 5.5 | 5.3 |

As is seen from the data in Table I, those compositions containing the alkoxylated alkyl phosphate ester showed significant improvement in spreadability as compared to those containing an alkoxylated alkyl ester with no phosphate.

## Claims

1. A composition useful for treating a keratinous substrate comprising:
(a) at least one alkoxylated alkyl phosphate ester;
(b) at least one ester oil;
(c) optionally, at least one non-ester oil component, such composition being substantially anhydrous.

2. The composition of claim 1, wherein the at least one alkoxylated alkyl phosphate ester corresponds to the general formula:
R₁ - (OCH₂ CH₂) ₙ(OCH(CH₃)CH₂)ₘ - OPO-(OH)₂
wherein R₁ is an alkyl group having from 8 to 40 carbon atoms, n is a number from 1 to 40, and m is a number from 0 to 20.

3. The composition of claim 2, wherein R₁ has from 14 to 18 carbon atoms.

4. The composition of any preceding claim, wherein the at least one alkoxylated alkyl phosphate ester is polypropylene glycol-5-ceteth-10 phosphate.

5. The composition of any preceding claim, wherein the at least one alkoxylated alkyl phosphate ester is present in the composition in an amount of from greater than 0 to about 10% by weight, based on the weight of the composition.

6. The composition of claim 5 wherein the at least one alkoxylated alky phosphate ester is present in the composition in an amount of from greater than 0 to about 3% by weight, based on the weight of the composition.

7. The composition of any preceding claim, wherein the at least one ester oil comprises at least one component chosen from mono-, di- and tri- esters of glycerol.

8. The composition of claim 7 wherein the at least one ester oil comprises at least one triglyceride.

9. The composition of claim 8 wherein the at least one ester oil comprises a caprylic/capric triglyceride.

10. The composition of any preceding claim, wherein the at least one ester oil component is present in the composition in an amount of from greater than 0 to about 95% by weight, based on the weight of the composition.

11. The composition of any preceding claim, wherein such composition comprises at least one non-ester oil component chosen from:
- linear, branched and/or cyclic alkanes;
- aliphatic fatty liquid monoalcohols containing 6 to 40 carbon atoms, the hydrocarbon-based chain not comprising a substitution group;
- volatile and/or non-volatile, optionally branched silicone oils;
- and mixtures thereof.

12. The composition of any preceding claim, wherein the at least one non-ester oil component is present in the composition in an amount of up to about 95% by weight, based on the weight of the composition.

13. The composition of claim 12, wherein the at least one non-ester oil component is present in the composition in an amount of up to about 50% by weight, based on the weight of the composition.

14. The composition of any preceding claim, wherein the composition is substantially free of mineral oil.

15. A process for treating a keratinous substrate comprising contacting the keratinous substrate with a composition as defined in any one of claims 1 to 14.
